# EUROPÄISCHE PATENTSCHRIFT

(11) **EP 2 663 539 B1**
(45) Veröffentlichungstag und Bekanntmachung des Hinweises auf die Patenterteilung: **27.07.2016**
(21) Anmeldenummer: 12701082.5
(22) Anmeldetag: 10.01.2012
(51) Int. Cl.: C07C 9/08, C07C 211/15, C07C 209/08

(54) **VERFAHREN ZUR HERSTELLUNG VON 2,2-DIFLUORETHYLAMIN AUS 2,2-DIFLUOR-1-CHLORETHAN UND AMMONIAK**
METHOD FOR PRODUCING 2,2-DIFLUOROETHYLAMINE FROM 2,2-DIFLUORO-1-CHLOROETHANE AND AMMONIA
PROCÉDÉ DE FABRICATION DE 2,2-DIFLUOROÉTHYLAMINE À PARTIR DE 2,2-DIFLUORO-1-CHLORÉTHANE ET AMMONIAC

(30) Priorität: 13.01.2011 EP 11150885; 02.02.2011 US 201161438679 P
(43) Veröffentlichungstag der Anmeldung: 20.11.2013
(73) Patentinhaber: Bayer Intellectual Property GmbH, 40789 Monheim (DE)
(72) Erfinder: LUI, Norbert, 51519 Odenthal (DE); WARSITZ, Rafael, 45136 Essen (DE); FUNKE, Christian, 42799 Leichlingen (DE); SEVERINS, Christian, 51379 Leverkusen (DE)
(74) Vertreter: BIP Patents
(86) Internationale Anmeldenummer: PCT/EP2012/050280
(87) Internationale Veröffentlichungsnummer: WO 2012/095403

(56) Entgegenhaltungen:
- WO-A1-2011/012243
- US-A- 4 030 994
- SWARTS: "Über einige fluorhaltige Alkylamine", CHEM. ZENTRALBLATT, Bd. 75, 1904, Seiten 944-945, XP009126801, in der Anmeldung erwähnt
- DICKEY ET AL.: INDUSTRIAL AND ENGINEERING CHEMISTRY, 1956, Seiten 209-213, XP002559402, in der Anmeldung erwähnt

## Beschreibung

Die vorliegende Erfindung betrifft ein Verfahren zur Herstellung von 2,2-Difluorethylamin ausgehend von 2,2-Difluor-1-chlorethan und Ammoniak.

2,2-Difluorethylamin ist eine wichtige Zwischenverbindung bei der Wirkstoffherstellung. Die Herstellung von 2,2-Difluorethylamin (sowie Tetrafluorethylamin) unter Verwendung einer Halogen-2,2-difluorethan-Verbindung, nämlich Brom-2,2-difluorethan, ist erstmals 1904 von Swarts in einem Artikel mit dem Titel "Über einige fluorhaltige Alkylamine" (Chem. Zentralblatt, Band 75, 1904, Seiten 944-945) beschrieben. Swarts verwendet 1-Brom-2,2-difluorethan und erhitzt dieses 3 Tage lang im Rohr mit 2 Mol alkoholischem Ammoniak auf 125-145 °C. Swarts beschreibt die vollständige Umwandlung der Ausgangsverbindung in die Verbindungen Difluorethylamin und Tetrafluorethylamin. Dem Artikel ist zu entnehmen, dass 2,2-Difluorethylamin nicht selektiv hergestellt wird, weil durch die Reaktion gleichermaßen Tetrafluorethylamin entstand, das abgetrennt werden muss. Beide Produkte wurden durch fraktionierte Destillation oder durch Umwandlung derselben in Chlorhydrate oder Oxalate isoliert.

Mehr als 50 Jahre später, nämlich 1956, veröffentlichte Dickey et al. in Industrial and Engineering Chemistry 1956, Nr. 2, 209- 213, ein Verfahren zur Herstellung von 2,2-Difluorethylamin, das von 2,2-Difluor-1-chlorethan und 28 %-igem Ammoniumhydroxid, d.h. 28 %-iger wässriger Ammoniak-Lösung ausgeht. Die Reaktanden werden in einem Autoklaven (rocking autoclave) zur Reaktion gebracht. Die Reaktionsmischung wird für 31 Stunden auf Temperaturen von 135 °C bis 140 °C erhitzt. Nach Beendigung der Reaktion wird die Reaktionsmischung filtriert und das Amin vom Reaktionsgemisch abdestilliert. Da sich aber noch eine Menge Ammoniak und etwas Wasser im Destillat befindet, wird das Amin über Natriumhydroxid getrocknet und nochmals destilliert.

Die bis 1956 veröffentlichten Verfahren haben den Nachteil, dass sie nicht selektiv sind, sehr lange dauern, und dass die Ausbeute eher gering ist. Wässriger Ammoniak in Kombination mit den in der Reaktionsmischung vorhandenen Chlorid- und Fluoridionen und in Kombination mit den in den Reaktionen verwendeten hohen Temperaturen ist stark korrodierend und greift metallische Werkstoffe an. Die Verfahren sind daher nicht geeignet, das gewünschte 2,2-Difluorethylamin in ausreichender Menge und kostengünstiger und umweltschonender Verwendung von Ressourcen (z.B. Energie und Ausgangsstoffe) herzustellen.

Seitdem sind keine Verfahren mehr veröffentlicht, die zur Herstellung von 2,2-Difluorethylamin eine Halogen-2,2-difluorethan-Verbindung, insbesondere 2,2-Difluor-1-chlorethau und Ammoniak verwenden.

In der internationalen Veröffentlichung WO2011/01224 (angemeldet als PCT/EP2010/004434) ist erstmals wieder ein Verfahren zur Herstellung von 2,2-Difluorethylamin beschrieben, das von 2,2-Difluor-1-halogenethan, vorzugsweise 2,2-Difluor-1-chlorethan, und Ammoniak ausgeht. Das Verfahren wird in Gegenwart eines Lösungsmittels durchgeführt, wobei der Wassergehalt des Lösungsmittel nur maximal 15 Vol.-% sein darf. Das Lösungsmittel wird in der Reaktion in 1- bis 50-facher Menge, wobei die 2- bis 20-fache Menge bevorzugt ist, eingesetzt. Obwohl das in der Patentanmeldung beschriebene Verfahren deutliche Vorteile gegenüber den 1904 und 1956 beschriebenen Verfahren aufweist, ist dieses Verfahren insbesondere in Bezug auf die umweltschonende Verwendung von Ressourcen verbesserungswürdig.

Ausgehend von den veröffentlichten und unveröffentlichten Verfahren zur Herstellung von 2,2-Difluorethylamin stellt sich nun die Aufgabe, ein Verfahren zu entwickeln, das von 2,2-Difluor-1-chlorethan und Ammoniak ausgeht und die oben genannten Nachteile vermeidet.

Es wurde überraschend gefunden, dass 2,2-Difluor-1-chlorethan und Ammoniak (NH₃) sich selektiv zu 2,2-Difluorethylamin umsetzen, selbst wenn kein Lösungsmittel vorhanden ist. Gleichfalls wurden Methoden gefunden, um die Reaktion kontinuierlich oder teilweise diskontinuierlich durchzuführen. Ein kontinuierliches bzw. teilweise diskontinuierliches Verfahren ist besonders bei der großtechnischen Herstellung von 2,2-Difluorethylamin vorteilhaft.

Gegenstand der Erfindung ist somit ein Verfahren zur Herstellung von 2,2-Difluorethylamin in Reinsubstanz, d.h. ohne Lösungsmittel, das die Umsetzung von 2,2-Difluor-1-chlorethan mit Ammoniak umfasst. Die Umsetzung kann gegebenenfalls in Gegenwart eines die Umsetzung mit Ammoniak beschleunigenden Katalysators erfolgen. Das Verfahren kann weiterhin einen Aufreinigungsschritt umfassen.

Die Anmeldung betrifft demzufolge ein Verfahren zur Herstellung von 2,2-Difluorethylamin, umfassend die folgenden Schritte:
(i) Vermischen von 2,2-Difluor-1-chlorethan und gasförmigen, flüssigen oder superkritischen Ammoniak in einem druckstabilen geschlossenen Reaktionsgefäß bei einem Druck im Bereich von 10 bis 180 bar; bevorzugt bei einem Druck im Bereich von 30 bar bis 155 bar;
(ii) Umsetzen der Reaktionsmischung bei einer Reaktionstemperatur im Bereich von 80 °C bis 200 °C; bevorzugt bei einer Reaktionstemperatur im Bereich von 100 °C bis 170 °C;
(iii) Entspannen der Reaktionsmischung und Abtrennen von 2,2-Difluorethylamin.

Im erfindungsgemäßen Verfahren wird kein Lösungsmittel verwendet.

In einer Ausführungsform [A] betrifft die Anmeldung ein Verfahren wie oben definiert, in dem in Schritt (i) noch zusätzlich ein Katalysator anwesend ist. Bevorzugt ist der Katalysator ausgewählt aus einer Gruppe bestehend aus Alkalibromiden, Alkalijodiden, Ammoniumbromid, Ammoniumjodid, Tetraalkylammoniumbromiden, Tetraalkylammoniumjodiden, Tetraalkylphosphoniumhalogeniden, Tetraarylphosphoniumhalogeniden Tetrakis(dimethylamino)phosphoniumbromid, Tetrakis(diethylamino)phosphoniumbromid, Tetrakis(dipropylamino)phosphoniumbromid, Tetrakis(dipropylamino)phosphoniumchlorid und Bis(dimethylamino)[(1,3-dimethylimidazolidin-2-yliden)amino]methyliumbromid. Besonders bevorzugte Katalysatoren sind Natriumbromid, Kaliumbromid, Natriumjodid, Kaliumjodid, Tetrabutylamoniumbromid oder Tetraphenylphosponiumbromid, ein ganz besonders bevorzugter Katalysator ist Natrium- oder Kaliumjodid.

In einer Ausführungsform [B] betrifft die Anmeldung ein Verfahren wie oben definiert bzw. wie in Ausführungsform [A] beschrieben, in dem das Zugeben/Zuführen von 2,2-Difluor-1-chlorethan und Ammoniak in Schritt (i) kontinuierlich erfolgt und in dem zum Vermischen ein statischer Mischer verwendet wird.

In einer Ausführungsform [C] betrifft die Anmeldung ein Verfahren wie oben definiert bzw. wie in Ausführungsform [A] oder [B] beschrieben, das vollständig kontinuierlich durchgeführt wird.

In einer Ausführungsform [D] betrifft die Anmeldung ein Verfahren wie in Ausführungsform [C] definiert, in dem als Reaktionsgefäß ein temperierbarer Durchflussreaktor verwendet wird, der mindestens zwei Zonen umfasst, wobei die erste Zone einen statischen Mischer beinhaltet und wobei Schritt (i) in dieser ersten Zone und Schritt (ii) in der zweiten Zone (Reaktionszone) erfolgt. Vorzugsweise umfasst der Durchflussreaktor noch eine weitere Zone am Ende der Reaktionszone oder anschließend an die Reaktionszone, in der der Reaktionsdruck reduziert wird.

In einer Ausführungsform [E] betrifft die Anmeldung ein Verfahren wie in Ausführungsform [D] definiert, in dem die Verweilzeit von 2,2-Difluor-1-chlorethan und Ammoniak in der zweiten Zone, d.h. der Reaktionszone, im Bereich von etwa 20 Sekunden bis etwa 400 Minuten; bevorzugt im Bereich von etwa 1 Minute bis etwa 400 Minuten; ganz besonders bevorzugt im Bereich von etwa 15 Minuten bis etwa 45 Minuten liegt.

In einer Ausführungsform [F] betrifft die Anmeldung ein Verfahren wie in Ausführungsfonn [D] oder [E] beschrieben, wobei im Durchflussreaktor, bevorzugt in der Reaktionszone, Messinstrumente oder Messsonden vorhanden sind.

Im erfindungsgemäßen Verfahren liegt das molare Verhältnis von 2,2-Difluor-1-chlorethan zum eingesetzten Ammoniak im Bereich von etwa 6 : 1 bis etwa 1 : 200, bevorzugt im Bereich von etwa 4 : 1 bis etwa 1 : 150, besonders bevorzugt im Bereich von etwa 1 : 1 bis etwa 1 : 100.

Der im erfindungsgemäßen Verfahren verwendete Ammoniak (NH₃) kann in gasförmigem, flüssigem (d.h. als kondensiertes NH₃-Gas), oder superkritischem Zustand (d.h. oberhalb seiner kritischen Temperatur und oberhalb seines kritischen Drucks) zum 2,2-Difluor-1-chlorethan zugegeben werden. Vorzugsweise erfolgt die Zugabe von NH₃ in Schritt (ii) in gasförmigem oder flüssigem Zustand.

Für die Verwendung im erfindungsgemäßen Verfahren sind alle Katalysatoren geeignet, die die Umsetzung mit Ammoniak beschleunigen. Mischungen geeigneter Katalysatoren sind auch denkbar. Erfindungsgemäß geeignete Katalysatoren sind insbesondere Alkalibromide und -jodide (z.B. Natriumjodid, Kaliumjodid, Kaliumbromid); Ammoniumbromid und Ammoniumjodid; Tetraalkylammoniumbromide und -jodide (z.B. Tetraethylammoniumjodid); bestimmte Phosphoniumhalogenide, wie Tetraalkyl- und Tetraarylphosphoniumhalogenide (z.B. Hexadecyl-tri-butyl-phosphoniumbromid, Stearyltributylphosphoniumbromid, Tetrabutylphosphoniumbromid, Tetraoctylphosphoniumbromid, Tetraphenylphosphoniumchlorid und Tetraphenylphosphoniumbromid), Tetrakis(dimethylamino)phosphoniumbromid, Tetrakis(diethylamino)phosphoniumbromid, Tetrakis(dipropylamino)phosphoniumchlorid und Tetrakis(dipropylamino)phosphoniumchlorid, bzw. -bromid; sowie Bis(dimethylamino)[(1,3-dimethylimidazolidin-2-yliden)amino]methyliumbromid.

Bevorzugte Katalysatoren sind Natriumbromid, Kaliumbromid, Natriumjodid, Kaliumjodid, Tetrabutylamoniumbromid oder Tetraphenylphosponiumbromid, besonders bevorzugt ist Natrium- oder Kaliumjodid.

Der Katalysator kann auch in-situ erzeugt werden. Beispielsweise durch eine Reaktion von HBr oder HJ mit Ammoniak. Weiterhin kann der Katalysator auch *in-situ* durch Zugabe von sehr reaktiven Alkylbromiden oder -jodiden (z.B. Methyl- oder Ethylbromid oder -jodid) erzeugt werden.

Im erfindungsgemäßen Verfahren wird der Katalysator, falls anwesend, bezogen auf das eingesetzte 2,2-Difluor-1-chlorethan, in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.-%. verwendet. Der Katalysator kann aber auch bevorzugt in einer Konzentration von etwa 0,05 bis etwa 3 Gew.-%, von etwa 0,1 bis etwa 10 Gew.-% oder von etwa 0,5 bis etwa 10 Gew.-%. eingesetzt werden.

Die Umsetzung wird grundsätzlich unter Druck in einem druckstabilen geschlossenen Reaktionsgefäß (z.B. Autoklav, Durchflussreaktor oder Rohrreaktor) durchgeführt. Der Druck während der Reaktion ist abhängig von der Menge an 2,2-Difluor-1-chlorethan, der Menge an verwendetem Ammoniak, der Reaktionstemperatur und, falls Pumpen zum Beladen des Reaktionsgefäßes verwendet werden, dem Förderdruck der Pumpen. Ist eine Druckerhöhung gewünscht, so kann beim diskontinuierlichen Verfahren neben dem Erhöhen der Reaktionstemperatur eine zusätzliche Druckerhöhung durch Zuleiten eines Inertgases, wie z.B. Stickstoff oder Argon, erreicht werden. Beim kontinuierlichem Verfahren kann der Druck durch die Pumpenleistung verändert werden.

Das Vermischen beider Edukte erfolgt bei einem Druck, der im Bereich von etwa 10 bis etwa 180 bar, bevorzugt im Bereich von 30 bis 155 bar, liegen sollte, wobei die Temperatur bei dem die Edukte vermischt werden variieren kann und gewöhnlich im Bereich von Raumtemperatur (etwa 20 °C) bis etwa 180 °C liegt. Die erfindungsgemäße Umsetzung kann in einem weiten Temperaturbereich (z.B. im Bereich von etwa 80 °C bis etwa 200 °C) durchgeführt werden. Vorzugsweise wird die Umsetzung in einem Temperaturbereich von etwa 100 °C bis etwa 170 °C durchgeführt.

Die Erfinder haben festgestellt, dass durch das Arbeiten in Substanz, d.h. ohne Zugabe eines Lösungsmittels, das verwendete Reaktionsgefäß (hier der Autoklav bzw. das Durchflussreaktor) trotz des hohen Reaktionsdruckes und trotz der verhältnismäßig hohen Reaktionstemperatur nur sehr gering korrodiert, mit der Konsequenz, dass das Verfahren im großtechnischen Maßstab durchgeführt werden kann.

Gleichfalls haben die Erfinder festgestellt, dass das gewünschte 2,2-Difluorethylamin mit dem erfindungsgemäßen Verfahren mit guten Ausbeuten, kurzen Reaktionszeiten und in hoher Reinheit erhalten wird, sodass eine umfangreiche Aufarbeitung des unmittelbaren Reaktionsprodukts im Allgemeinen nicht erforderlich ist. All das ist überraschend, denn aus den M. Hudlicky, Chemistry of Organofluorine Compounds, 2nd Editon, 1976, p. 489-490 und Houben Weyl, E 10b/2 p. 92-98 ist bekannt, dass unter basischen Bedingungen sich bevorzugt das Vinylidenfluorid bildet, das unter Abspaltung von HCl aus 2,2-Difluorchlorethan entstehen kann. Auch ist aus J. Org. Chem. 2007, 72 (22) 8569 bekannt, dass 2,2-Difluorethylamin sehr reaktiv ist und unter den erfindungsgemäßen Reaktionsbedingungen weiterreagieren kann.

Das erfindungsgemäße Verfahren kann sowohl vollständig kontinuierlich als auch vollständig diskontinuierlich durchgeführt werden. Gleichfalls können in dem diskontinuierlich geführten erfindungsgemäßen Verfahren einige Reaktionsschritte kontinuierlich durchgeführt werden. Solche Verfahren werden "teilweise diskontinuierlich" genannt.

Unter kontinuierlichem Verfahren oder Schritten werden hier solche verstanden, in denen der Zulauf der Edukte in ein Reaktionsgefäß (z.B. Durchflussreaktor) und der Austrag der Produkte aus dem Reaktionsgefäß gleichzeitig, jedoch räumlich getrennt stattfinden. Unter diskontinuierlichem Verfahren oder Schritten werden hier solche verstanden, die zeitlich nacheinander ablaufen, z.B. Zulauf der Edukte in ein Reaktionsgefäß, dann Umsetzung der Edukte gefolgt vom Austrag der Produkte aus dem Reaktionsgefäß. Das kontinuierliche Verfahren ist insbesondere zum Herstellen großer Mengen geeignet.

Die Reaktionsdauer des teilweise oder vollständig diskontinuierlich durchgeführten erfindungsgemäßen Verfahrens liegt im Bereich von etwa 0,5 bis etwa 16 Stunden. Eine längere Reaktionsdauer ist grundsätzlich möglich.

Es ist bevorzugt, das Zugeben und das Vermischung des Schritts (i) des erfindungsgemäßen Verfahrens kontinuierlich durchzuführen, wobei ein statischer Mischer verwendet wird.

Im kontinuierlich durchgeführten erfindungsgemäßen Verfahren wird in einem Durchflussreaktor gearbeitet. Der Durchflussreaktor ist vorzugsweise temperierbar. Ein erfindungsgemäß geeigneter Durchflussreaktor umfasst mindestens zwei Zonen, wovon mindestens eine der Zonen temperierbar ist. In der ersten Zone erfolgt das Vermischen der Edukte, nämlich 2,2-Difluor-1-chlorethan und NH₃. Wird in der erfindungsgemäßen Reaktion ein Katalysator verwendet, dann kann er zusammen mit einem der Edukte, insbesondere 2,2-Difluor-1-chlorethan, in das Reaktionsgefäß eingebracht werden. In der ersten Zone ist vorteilhafterweise ein statischer Mischer eingebaut. In der zweiten, temperierbaren Zone, der sogenannten Verweil- oder Reaktionszone, erfolgt die Umsetzung. Am Ende der Reaktionszone wird der Reaktionsdruck reduziert, wodurch gelöstes NH₃ teilweise oder vollständig verdampft. Durch die Verminderung des Drucks kristallisieren die während der Reaktion gebildeten organischen und/oder anorganischen Salze teilweise oder vollständig aus. Flüssiges 2,2-Difluorethylamin kann dann aufgefangen und gesammelt werden.

Die gewünschten Verweilzeiten in der Verweil- bzw. Reaktionszone bzw. die zu erreichenden Reaktionsumsätze hängen in erster Linie von der Dosiergeschwindigkeit aller Reaktionspartner einschließlich gegebenenfalls anwesenden Katalysators (Reaktionsmischung), und der Durchflussrate der Reaktionsmischung durch die Verweilstrecke (Länge der Reaktionszone) ab. Allgemein gilt, dass die Länge der Verweilzeit von der Reaktionstemperatur abhängt: Je höher die Reaktionstemperatur, desto kürzer sollte die Verweilzeit sein. In der Regel liegen die Verweilzeiten der Reaktionsmischung in der Reaktionszone im Bereich von etwa 20 Sekunden (sek.) bis etwa 400 Minuten (min.), bevorzugt im Bereich von etwa 1 min. bis etwa 400 min., ganz besonders bevorzugt im Bereich von etwa 15 min. bis etwa 45 min.

Es ist denkbar, der Reaktionsmischung entlang der gesamten oder eines Teils der Verweilstrecke NH₃ zuzuführen.

Die Verweilzeit der Reaktionsmischung in der Reaktionszone wird normalerweise durch die Durchflußrate durch die Reaktionszone und das Volumen der Reaktionszone gesteuert. Es ist von Vorteil, wenn der Reaktionsverlauf durch Messinstrumente, wie z.B. Temperaturmesser, Viskosimeter, Wärmeleitfähigkeitsmesser oder Refraktometer oder Instrumente oder Sonden zur Messung von Infrarot- und/oder Nahinfrarotspektren, verfolgt wird. Die Messinstrumente müssen natürlich für strömende Medien geeignet sein und können in den Durchflussreaktor integriert sein.

Statische Mischer sind in der Regel Rohre oder Kanäle mit feststehenden Einbauten, die unter Nutzung der Strömungsenergie die Mischung fluider Produktströme bewirken. Eine Fördereinheit (z.B. eine Pumpe) drückt die Flüssigkeit z.B. durch das mit feststehenden (=statischen) Mischereinbauten versehene Rohr. Hierbei werden die der Hauptströmungsachse folgenden fluiden Produktströme in Teilströme aufgeteilt, die dann - je nach Art der Einbauten - miteinander verwirbelt und vermischt werden.

Gegenüber Mischern mit bewegten Rührorganen haben statische Mischer ein oftmals kleineres Kammervolumen und einen sehr geringen Leistungsbedarf. Statische Mischer sind geschlossene Systeme die weder Wartung noch Inertisierung brauchen. Bei statischen Mischern wird die Strömungsenergie der fluiden Produktströme ausgenutzt, währen bei dynamischen Mischern (z.B. Rührer) das Vermischen bzw. die Homogenisierung der zu mischenden fluiden Produktströme durch bewegte Organe erreicht wird.

Einen Überblick über verschiedene Typen statischer Mischer, wie sie in der konventionellen Verfahrenstechnik eingesetzt werden, gibt zum Beispiel der Artikel "Statische Mischer und ihre Anwendungen" von M. H. Pahl und E. Muschelknautz, in Chem.-Ing.-Techn. 52 (1980) Nr. 4, S. 285-291. Auf diesen Artikel und die darin beschriebenen statischen Mischer wird hiermit ausdrücklich Bezug genommen. Statische Mischer sind beispielsweise in US 4,062,524 B beschrieben und sind unter dem Handelsnamen "Sulzer SMX^{®}" Mischer käuflich erhältlich. Die vorgenannten Mischer bestehen aus zwei oder mehr zueinander senkrecht stehenden Gittern paralleler Streifen, die an ihren Kreuzungspunkten miteinander verbunden sind und in einem Winkel gegen die Hauptströmungsrichtung der zu vermischenden fluiden Produktströme angestellt sind, um die Produktströme in Teilströme zu teilen und - wie oben beschrieben- zu mischen. Da die Durchmischung nur entlang einer Vorzugsrichtung quer zur Hauptströmungsrichtung erfolgt, müssen mehrere Mischereinbauten, die zueinander um 90° verdreht sind, hintereinander angeordnet sein.

Nach erfolgter Umsetzung im kontinuierlichen, teilweise diskontinuierlichen oder diskontinuierlichen Verfahren, wird die Reaktionsmischung vorzugsweise aufgearbeitet und das gewünschte 2,2-Difluorethylamin isoliert und, wenn nötig, gereinigt. Demzufolge umfasst Schritt (iii) des erfindungsgemäßen Verfahrens Entspannen der Mischung, d.h. Ablassen des im Reaktionsgefäß vorherrschenden Drucks, und Abtrennen des Katalysators, falls vorhanden, und der während der Reaktion entstandenen anorganischen und/oder organischen Salze. Die Abtrennung geschieht vorteilhafterweise durch Filtration der Reaktionsmischung und fraktionierter Destillation des 2,2-Difluorethylamins.

Durch Variation des Reaktionsdruckes können während der Reaktion gebildete organische und/oder anorganischen Salze, zum Beispiel Ammoniumchlorid, teilweise oder vollständig auskristallisiert werden. Durch das Entspannen, wird nicht verbrauchtes, noch gelöstes Ammoniak teilweise oder vollständig entfernt.

Die Isolierung von 2,2-Difluorethylamin erfolgt mit bekannten Methoden, nämlich Extraktion, (fraktionierter) Destillation, Chromatographie, wobei die Methoden kombiniert werden können. Die Isolierung oder Aufreinigung eines 2,2-Difluorethylamin-Salzes, beispielsweise Salze organischer oder anorganischer Säuren (z.B. Hydrochloride oder Acetate), erfolgt bevorzugt durch Kristallisation. 2,2-Difluorethylamin-Salze sind z.B. das 2,2-Difluorethylamin-Hydrochlorid oder 2,2-Difluorethylamin-Acetat. Wasserlösliche Salze können durch Extraktion der wässrigen Lösungen gereinigt werden. Das Amin kann dann schließlich durch Umsetzung mit organischen oder anorganischen Basen aus seinen Salzen freigesetzt werden. Bevorzugte Basen sind NaHCO₃, Na₂CO₃ oder NaOH.

Die vorliegende Erfindung wird anhand der nachfolgenden Beispiele näher erläutert, ohne die Erfindung dabei auf diese einzuschränken.

### Herstellungsbeispiele der Umsetzung von 2,2-Difluor-1-chlorethan und Ammoniak:

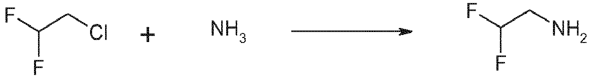

### Beispiel 1 - diskontinuierliches Verfahren

215 g (2,10 mol) 2,2-Difluor-1-chlorethan, 2,38 g Kaliumbromid werden in einem Autoklaven vorgelegt und mit 136 g Ammoniak (wasserfrei) versetzt. Das molare Verhältnis von 2,2-Difluor-1-chlorethan zu Ammoniak beträgt 1 : 4. Die Reaktionsmischung wird auf 140-145°C erhitzt und 9 Stunden bei dieser Temperatur gerührt. Bei der Reaktion nimmt der Druck von etwa 50 bar auf etwa 35 bar ab. Die Reaktionsmischung wird auf 0°C abgekühlt und innerhalb von 1 Stunde entspannt. Es wird mit 400 g N-Methylpyrrolidon (NMP) und Wasser versetzt bis alle Salze gelöst sind. Nach quantitativer GC-Analyse (externer Standard) erhält man eine chemische Ausbeute von 59% an 2,2-Difluorethylamin bezogen auf eingesetztes 2,2-Difluor-1-chlorethan.

### NMR ¹H (CDCl₃) : 5,5 - 5,9 (m, 1H), 2,94 - 3,1 (m, 2 H), 1,26 (br m, NH₂)

### Beispiel 2 - kontinuierliches Verfahren unter Verwendung eines Durchflussreaktors

2,2-Difluor-1-chlorethan (98%-ige Ware) wurde in eine Vorlage 1 eingefüllt. Vorlage 1 wird mit einer Hochdruckpumpe verbunden. Eine Ammonik-Gasflasche mit wasserfreien NH₃ wurde als Vorlage 2 mit einer weiteren Hochdruckpumpe verbunden. Die beiden Vorlagen wurden durch eine Vortemperierstrecke (Raumtemperatur) mit einem statischen Mischer, mit einem Volumen von 10 ml (Mischzone) verbunden, an dessen Auslasskanal ein temperierbares Verweilelement mit 57,2 cm³ Volumen (Reaktionszone) und einem Verhältnis von Oberfläche zu Volumen von 18 cm²/cm³ (bei 155°C), angeschlossen war. Am Ausgang der Verweilerstrecke war ein pneumatisch geregeltes Kämmerventil angebracht. Durch das Kämmerventil konnte der Druck im Reaktor konstant auf 155 bar gehalten werden und gleichzeitig in den nachgeschalteten Phasenseparator entspannt werden. 2,2-Difluor-1-chlorethan aus Vorlage 1 wurde mit einem Volumenstrom von 0,16 ml/min und Ammoniak aus Vorlage 2 wurde mit einem Volumenstrom von 1,28 ml/min kontinuierlich durch den Reaktor gepumpt. Die Verweilzeit der Reaktionsmischung in der Mischzone betrug 7 min und die Verweilzeit in der Reaktionszone betrug 40 min. Die Reaktion wurde per HPLC verfolgt.

Der Produktstrom wurde hinter dem Kämmerventil in einen Phasenseparator entspannt, dabei kristallisierte durch die Reaktion gebildetes NH₄Cl aus. Nach Phasentrennung und Filtration wurde 2,2-Difluorethylamin gesammelt. Nach quantitativer Gaschromatographischer -Analyse (externer Standard) wird eine chemische Ausbeute von 60-70% an 2,2-Difluorethylamin bezogen auf eingesetztes 2,2-Difluor-1-chlorethan erhalten.

NMR ¹H (CDCl₃) *:* 5,5 - 5,9 (m, 1H), 2,94 - 3,1 (m, 2 H), 1,26 (br m, NH₂)

## Patentansprüche

1. Verfahren zur Herstellung von 2,2-Difluorethylamin, umfassend die folgenden Schritte:
(i) Vermischen von 2,2-Difluor-1-chlorethan und gasförmigen, flüssigen oder superkritischen Ammoniak in einem druckstabilen geschlossenen Reaktionsgefäß bei einem Druck im Bereich von 10 bis 180 bar;
(ii) Umsetzen der Reaktionsmischung bei einer Reaktionstemperatur im Bereich von 80 °C bis 200 °C;
(iii) Entspannen der Reaktionsmischung und Abtrennen von 2,2-Difluorethylamin,
wobei in dem Verfahren kein Lösungsmittel verwendet wird.

2. Verfahren nach Anspruch 1, in dem in Schritt (i) noch zusätzlich ein Katalysator anwesend ist, der ausgewählt aus einer Gruppe bestehend aus Alkalibromiden, Alkalijodiden, Ammoniumbromid, Ammoniumjodid, Tetraalkylammoniumbromiden, Tetraalkylammoniumjodiden, Tetraalkylphosphoniumhalogeniden, Tetraarylphosphoniumhalogeniden Tetrakis(dimethylamino)-phosphoniumbromid, Tetrakis(diethylamino)phosphoniumbromid, Tetrakis(dipropylamino)-phosphoniumbromid, Tetrakis(dipropylamino)phosphoniumchlorid und Bis(dimethylamino)[(1,3-dimethylimidazolidin-2-yliden)amino]methyliumbromid.

3. Verfahren nach Anspruch 1 oder 2 in dem in Schritt (i) das Zugeben von 2,2-Difluor-1-chlorethan und Ammoniak kontinuierlich erfolgt und zum Vermischen ein statischer Mischer verwendet wird.

4. Verfahren nach einem der Ansprüche 1 bis 3 das vollständig kontinuierlich durchgeführt wird.

5. Verfahren nach Anspruch 4, in dem als Reaktionsgefäß ein temperierbarer Durchflussreaktor verwendet wird, der mindestens zwei Zonen umfasst, wobei die erste Zone einen statischen Mischer beinhaltet und wobei Schritt (i) in dieser ersten Zone und Schritt (ii) in der zweiten Zone (Reaktionszone) erfolgt. Vorzugsweise umfasst der Durchflussreaktor noch eine weitere Zone am Ende der Reaktionszone oder anschließend an die Reaktionszone, in der der Reaktionsdruck reduziert wird.

6. Verfahren nach Anspruch 4, in dem die Verweilzeit von 2,2-Difluor-1-chlorethan und Ammoniak in der zweiten Zone, d.h. der Reaktionszone, im Bereich von etwa 20 Sekunden bis etwa 400 Minuten liegt.

7. Verfahren nach Anspruch 5 oder 6, wobei im Durchflussreaktor Messinstrumente oder Messsonden vorhanden sind.

## Claims

1. Process for preparing 2,2-difluoroethylamine, comprising the following steps:
(i) mixing 2,2-difluoro-1-chloroethane and gaseous, liquid or supercritical ammonia in a pressure-stable, closed reaction vessel under a pressure in the range from 10 to 180 bar;
(ii) reacting the reaction mixture at a reaction temperature in the range from 80°C to 200°C;
(iii) letting down the reaction mixture and isolating 2,2-difluoroethylamine,
no solvent being used in the process.

2. Process according to Claim 1, in which a catalyst is additionally present in step (i), said catalyst being selected from a group consisting of alkali metal bromides, alkali metal iodides, ammonium bromide, ammonium iodide, tetraalkylammonium bromides, tetraalkylammonium iodides, tetraalkylphosphonium halides, tetraarylphosphonium halides, tetrakis(dimethylamino)phosphonium bromide, tetrakis(diethylamino)phosphonium bromide, tetrakis(dipropylamino)phosphonium bromide, tetrakis(dipropylamino)phosphonium chloride, and bis(dimethylamino)[(1,3-dimethylimidazolidin-2-ylidene)amino]methylium bromide.

3. Process according to Claim 1 or 2, in which in step (i) the addition of 2,2-difluoro-1-chloroethane and ammonia takes place continuously and a static mixer is used for the mixing.

4. Process according to any of Claims 1 to 3, carried out completely continuously.

5. Process according to Claim 4, using as reaction vessel a thermally conditionable flow reactor which comprises at least two zones, the first zone comprising a static mixer, and step (i) taking place in this first zone and step (ii) taking place in the second zone (reaction zone). The flow reactor preferably further comprises an additional zone at the end of the reaction zone or subsequent to the reaction zone, in which the reaction pressure is reduced.

6. Process according to Claim 4, in which the residence time of 2,2-difluoro-1-chloroethane and ammonia in the second zone, i.e. the reaction zone, is in the range from about 20 seconds to about 400 minutes.

7. Process according to Claim 5 or 6, where measuring instruments or measuring probes are present in the flow reactor.

## Revendications

1. Procédé de fabrication de 2,2-difluoroéthylamine, comprenant les étapes suivantes :
(i) le mélange de 2,2-difluoro-1-chloroéthane et d'ammoniac gazeux, liquide ou supercritique dans un récipient de réaction fermé de pression stable à une pression dans la plage allant de 10 à 180 bar ;
(ii) la mise en réaction du mélange réactionnel à une température de réaction dans la plage allant de 80 °C à 200 °C ;
(iii) la détente du mélange réactionnel et la séparation de 2,2-difluoroéthylamine,
aucun solvant n'étant utilisé dans le procédé.

2. Procédé selon la revendication 1, selon lequel un catalyseur est en outre présent à l'étape (i), qui est choisi dans le groupe constitué par les bromures alcalins, les iodures alcalins, le bromure d'ammonium, l'iodure d'ammonium, les bromures de tétraalkylammonium, les iodures de tétraalkylammonium, les halogénures de tétraalkylphosphonium, les halogénures de tétraarylphosphonium, le bromure de tétrakis(diméthylamino)phosphonium, le bromure de tétrakis(diéthylamino)phosphonium, le bromure de tétrakis(dipropylamino)phosphonium, le chlorure de tétrakis(dipropylamino)phosphonium et le bromure de bis(diméthylamino)[(1,3-diméthylimidazolidin-2-ylidène)amino]méthylium.

3. Procédé selon la revendication 1 ou 2, selon lequel, à l'étape (i), l'ajout de 2,2-difluoro-1-chloroéthane et d'ammoniac a lieu en continu et un mélangeur statique est utilisé pour le mélange.

4. Procédé selon l'une quelconque des revendications 1 à 3, qui est réalisé entièrement en continu.

5. Procédé selon la revendication 4, selon lequel un réacteur en écoulement régulable en température est utilisé en tant que récipient de réaction, qui comprend au moins deux zones, la première zone contenant un mélangeur statique et l'étape (i) ayant lieu dans cette première zone et l'étape (ii) ayant lieu dans la deuxième zone (zone de réaction), le réacteur en écoulement comprenant de préférence encore une zone supplémentaire à la fin de la zone de réaction ou après la zone de réaction, dans laquelle la pression de réaction est réduite.

6. Procédé selon la revendication 4, selon lequel le temps de séjour du 2,2-difluoro-1-chloroéthane et de l'ammoniac dans la deuxième zone, c.-à-d. la zone de réaction, est dans la plage allant d'environ 20 secondes à environ 400 minutes.

7. Procédé selon la revendication 5 ou 6, selon lequel des instruments de mesure ou des sondes de mesure sont présents dans le réacteur en écoulement.
